# EUROPÄISCHE PATENTSCHRIFT

(11) **EP 2 184 038 B1**
(45) Veröffentlichungstag und Bekanntmachung des Hinweises auf die Patenterteilung: **25.11.2015**
(21) Anmeldenummer: 09174331.0
(22) Anmeldetag: 28.10.2009
(51) Int. Cl.: A61F 2/86, A61F 2/91, A61L 31/10, A61L 31/18

(54) **Endoprothese**
Endoprosthesis
Endoprothèse

(30) Priorität: 11.11.2008 DE 102008043642
(43) Veröffentlichungstag der Anmeldung: 12.05.2010
(73) Patentinhaber: Biotronik VI Patent AG, 6341 Baar (CH)
(72) Erfinder: Klocke, Björn, 8006, Zürich (CH); Riedmüller, Johannes, 90411, Nürnberg (DE)
(74) Vertreter: Galander, Marcus

(56) Entgegenhaltungen:
- EP-A1- 1 886 650
- WO-A1-01/58384
- WO-A1-2004/105642
- WO-A1-2008/101987
- US-A1- 2004 015 229

## Beschreibung

Die vorliegende Erfindung betrifft eine Endoprothese oder Implantat, insbesondere intraluminale Endoprothese, z.B. ein Stent, mit einem Grundkörper, der zumindest teilweise aus einem metallischen Material besteht, und mit einem an dem Grundkörper befestigten oder in dieses eingebetteten Funktionselement, das mindestens teilweise radioopakes und/oder röntgenopakes Material aufweist, das zumindest in einem Teil seines Volumens eine verglichen mit dem Material des Grundkörpers verschiedene Materialzusammensetzung aufweist.

Stents sind endovaskuläre Prothesen, die zur Behandlung von Stenosen (Gefäßverengungen) eingesetzt werden können. Sie weisen einen Grundkörper in Form eines hohlzylinder- oder röhrenförmigen Grundgitters auf, das an beiden Längsenden der Röhren offen ist. Das röhrenförmige Grundgitter einer derartigen Endoprothese wird in das zu behandelnde Gefäß eingesetzt und dient dazu, das Gefäß zu stützen.

Derartige Stents oder andere Endoprothesen weisen in ihrem Grundkörper häufig metallische Materialien auf. Hierbei können die metallischen Materialien einen biodegradierbaren Werkstoff bilden, wobei auch polymere, biodegradierbare Materialien enthalten sein können.

Unter Biodegradation werden hydrolytische, enzymatische und andere stoffwechselbedingte Abbauprozesse im lebenden Organismus verstanden, die vor allem durch die mit der Endoprothese in Kontakt gelangenden Körperflüssigkeiten verursacht werden und zu einer allmählichen Auflösung zumindest großer Teile der Endoprothese führen. Synonym zu dem Begriff Biodegradation wird häufig der Begriff Biokorrosion verwendet. Der Begriff Bioresorption umfasst die anschließende Resorption der Abbauprodukte durch den lebenden Organismus.

Für den Grundkörper biodegradierbarer Endoprothesen geeignete Werkstoffe (Grundma-terial) können auch aus mehreren Materialien bestehen. Beispiele für geeignete polymere Verbindungen sind Polymere aus der Gruppe Cellulose, Kollagen, Albumin, Casein, Polysaccharide (PSAC), Polylactid (PLA), Poly-L-Lactid (PLLA), Polyglykol (PGA), Poly-D,L-Lactid-co-glycolid (PDLLA-PGA), Polyhydroxybuttersäure (PHB), Polyhydroxyvaleriansäure (PHV), Polyalkylcarbonate, Polyorthoester, Polyethylenterephtalat (PET), Polymalonsäure (PML), Polyanhydride, Polyphosphazene, Polyaminosäuren und deren Copolymere sowie Hyaluronsäure. Die Polymere können je nach den gewünschten Eigenschaften in Reinform, in derivatisierter Form, in Form von Blends oder als Copolymere vorliegen. Metallische biodegradierbare Werkstoffe basieren beispielsweise auf Legierungen von Magnesium, Eisen, Zink und/oder Wolfram.

Die vorliegende Erfindung bezieht sich vorzugsweise auf Stents oder andere Endoprothesen mit einem Grundkörper, dessen Material einen metallischen Werkstoff enthält. Hierbei kommen neben den genannten biodegradierbaren Materialien als weitere metallische Werkstoffe Edelmetalle wie Platin, Iridium, Gold, Tantal, Yttrium, Zirkonium, Ytterbium oder deren Legierungen in Frage.

Es ist bekannt, Stents mit Funktionselementen zu versehen, die zumindest in einem Teil ihres Volumens eine verglichen mit dem Material des Grundkörpers verschiedene Materialzusammensetzung aufweisen. Diese Funktionselemente dienen beispielsweise der Bestimmung der Position eines Stents im Körper oder der Freisetzung von Medikamenten.

Die Ermittlung der Position eines Stents geschieht häufig mittels bildgebender Verfahren, beispielsweise mittels einer Röntgenstrahleinrichtung. Da die für den Grundkörper von Stents verwendeten Materialien in der Regel Röntgenstrahlung nur in geringem Maße absorbieren, d.h. röntgen- und/oder radioluzent sind, werden Stents häufig mit sogenannten Markern als Funktionselementen versehen, die ein Material enthalten, das die Röntgenstrahlen und/oder andere elektromagnetische Strahlen stärker absorbiert (im Folgenden als röntgenopakes oder radioopakes Material bezeichnet) als das Material des Grundkörpers.

In der Druckschrift US 6,355,058 B1 wird ein Stent beschrieben, bei dem radioopake Marker in einem polymeren Bindemittel als Partikel eingeschlossen sind. Der Binder ist auf der Oberfläche des Stents ausgebreitet (dispergiert). Eine derartige Verteilung von radioopaken Partikeln bewirkt in der Regel keine ausreichende Dichte dieser Materialien, so dass die Röntgensichtbarkeit für viele Anwendungen zu gering ist.

In der Druckschrift US 6,293,966 B1 wird ein Stent mit radioopaken Markerelementen offenbart, der an seinen distalen bzw. proximalen Enden C-förmig ausgebildete Elemente aufweist, die jeweils eine im Wesentlichen kugelförmige Aufnahme ausbilden. In diese Aufnahmen werden Markerelemente mit kugelförmigen Endabschnitten eingesetzt. Die kugelförmigen Endabschnitte werden formschlüssig und ggf. mittels einer Schweißverbindung in den durch die C-förmigen Elemente gebildeten Aufnahmen befestigt.

Die Druckschrift DE 698 36 656 T2 zeigt und beschreibt einen bioabsorbierbaren Marker mit röntgendichten Bestandteilen zur Verwendung auf einer implantierbaren Endoprothese, wie einem Stent. Die bioabsorbierbaren röntgendichten Marker weisen beispielsweise poröse Abschnitte auf, die mit röntgendichtem Material gefüllt sind. Außerdem wird ein Marker beschrieben, der hohle, hohlraumartige und poröse Abschnitte aufweist, die mit röntgendichtem Material gefüllt sind. Außerdem zeigt der Stand der Technik einen Marker, der als längliches Element wie einem Filament ausgebildet ist, welches um Teile der implantierbaren Endoprothese geschlungen wird.

Die Druckschrift US 2004/0015229 offenbart die technischen Merkmale aus dem Oberbegriff des Anspruchs 1.

Bei Stents, deren Grundkörper aus einem metallischen Material besteht, ergibt sich bei der Anordnung von Funktionselementen, beispielsweise bei radioopaken Elementen aus Gold oder Silber, an dem Stent-Grundkörper das Problem, dass an dem Kontaktbereich zwischen dem Material des Grundkörpers und dem Material des Funktionselements eine Kontaktkorrosion auftritt. Diese führt zur Zerstörung des Stents bzw. zur Abtrennung des Funktionselements von der Stentstruktur, so dass die Endoprothese nicht mehr in der Lage ist, ihre Funktion zu erfüllen, bzw. nicht mehr aufgefunden werden kann. Für das geschilderte Problem enthalten die oben angegebenen Endoprothesen aus dem Stand der Technik keine Lösung.

Die Aufgabe der vorliegenden Erfindung besteht somit darin, eine Endoprothese zu schaffen, bei der Funktionselemente mit einem metallischen Material nicht mehr die beschriebene Kontaktkorrosion aufweisen.

Die obige Aufgabe wird durch eine Endoprothese gelöst, bei der das Funktionselement mit einer Barriereschicht versehen ist, mittels der das radioopake und/oder röntgenopake Material gegenüber dem Grundkörper elektrisch isoliert ist.

Eine derartige erfindungsgemäße Endoprothese hat den Vorteil, dass sie trotz Kombination verschiedener Metalle keinen Batterieeffekt und damit keine Kontaktkorrosion bzw. bei degradierbaren Materialien keine ungewollten Degradation (oder Beschleunigung der Degradation) des Stents aufweist. Durch die Barriereschicht verbleibt das Funktionselement, das einen röntgen- oder radioopaken Marker darstellt, länger und unter Umständen permanent in dem behandelten Gefäß, so dass das Funktionselement auch noch zu einem späteren Zeitpunkt den Ort der Implantation der Endoprothese anzeigt, z.B. auch noch dann, wenn die Endoprothese bereits teilweise degradiert ist.

Bei der erfindungsgemäßen Endoprothese umschließt die Barriereschicht das Funktionselement vollständig. Hierdurch wird eine besonders effektive Isolierung des Funktionselements erreicht.

In einem weiteren Ausführungsbeispiel der Erfindung weist die Barriereschicht eine oder mehrere Materialien aus der Gruppe enthaltend Parylene, vorzugsweise Parylene C, Polyzene F, Silikon, Gycocalix, DLC (diamond-like carbon), Cyanacrylate (insbesondere Methyl-2-Cyanoacrylat, n-Butylcyanacrylat und 2-Octyl-Cyanarylat) und Titandioxid auf. Generell eignen sich auch weitere nicht-leitende, permanente hydrophobe Polymere. Die angegebenen Verbindungen sind besonders gut elektrisch isolierend und zeichnen sich außerdem durch eine geringe Wasserdurchlässigkeit, hohe Permanenz und Biokompatibilität aus. Diese Eigenschaften sind für die Anwendung als Endoprothese besonders vorteilhaft. Die Dicke der Barriereschicht liegt vorzugsweise zwischen etwa 1 µm und etwa 20 µm.

Hierbei werden als Parylene vollständig lineare, teilkristalline und unvernetzte aromatische Polymere bezeichnet. Diese Polymere lassen sich je nach ihrem Aufbau in vier verschiedene Grundtypen einteilen, nämlich Parylene C, Parylene D, Parylene N und Parylene F. Parylene kann auf das Funktionselement bevorzugt mittels eines plasmachemischen Beschichtungsverfahrens aufgebracht werden.

Das Funktionselement weist vorzugsweise eine oder mehrere röntgen- und/oder radioopake Elemente oder Verbindungen aus der Gruppe bestehend aus Platin, Iridium, Gold, Wolfram, Tantal, Yttrium, Zirkon, Ytterbium, Legierungen aus diesen Metallen und Jodverbindungen auf.

Um ein Volumen bzw. eine Ausdehnung des Funktionselements bereitzustellen, das eine ausreichende Röntgensichtbarkeit erzeugt, umschließt in einem bevorzugten Ausführungsbeispiel die Barriereschicht ein Volumen des röntgen- und/oder radioopaken Materials eines Funktionselements mit einer Querschnittsfläche von mindestens etwa 200 µm x etwa 100 µm. Für eine gute Röntgensichtbarkeit sollte idealerweise eine Ausdehnung der Querschnittsfläche in mindestens eine Dimension (Richtung) von mindestens etwa 300 µm realisiert sein. Dabei kann die genannte Querschnittsfläche oder Ausdehnung in einer Dimension auch durch zwei oder mehrere Funktionselemente, die entsprechend nahe beieinander angeordnet sind, realisiert werden. Die Ausdehnung bzw. das Volumen des Funktionselements soll dabei insbesondere so groß gewählt sein, dass die Ausdehnung bzw. das Volumen mindestens zwei Pixel der Auflösung gängiger Angiographie-Geräte umfasst.

Bevorzugt ist das Funktionselement mit der Barriereschicht mittels einer Formschluss-Verbindung und/oder einer Klebeverbindung mit dem Grundkörper verbunden. Diese Verbindungen sind besonders einfach herstellbar. Im Falle der Verwendung von Cyanacrylat in der Barriereschicht kann diese auch die Funktion der Klebeverbindung selbst erfüllen.

Wie oben bereits ausgeführt wurde, ist es bevorzugt, wenn der Grundkörper ein biodegradierbares Material enthält, vorzugsweise Magnesium und/oder eine Magnesiumlegierung.

Besonders einfach lassen sich Funktionselemente an dem Grundkörper dann befestigen, wenn diese zwei durchgehende Öffnungen zur Befestigung am Grundkörper aufweisen. Zusätzlich können die Funktionselemente eine umlaufende Nut zur Befestigung am Grundkörper vorsehen.

Auf einfache Weise lassen sich Funktionselemente ebenfalls an dem Grundkörper befestigen, wenn das Funktionselement mit der Barriereschicht drahtförmig ausgebildet ist. Solche Funktionselemente werden dann bevorzugt parallel zu einer Struktur des Grundkörpers angebracht.

Es ist weiterhin von Vorteil, wenn die Geometrie des Funktionselements und/oder die Verbindung des Funktionselements mit dem Grundkörper so gewählt wird, dass die Wirkung der Endoprothese, vorzugsweise des Stents, nach dem Crimpen, Track oder der Dilatation hinsichtlich der klinischen Stützwirkung nicht maßgeblich beeinträchtigt wird. Ebenso soll das vollständige Einwachsen in das behandelte Gefäß begünstigt werden (Neointima-Bildung). Hierbei darf der Fluss der Körperflüssigkeit nicht behindert werden. Das Funktionselement sollte deshalb in einem bevorzugten Ausführungsbeispiel nicht in das Stent-Lumen hineinragen. Zu diesem Zweck sollte das Funktionselement in mindestens einer Richtung einen nicht-runden bzw. nicht-ovalen Vollquerschnitt aufweisen, so dass ein Gefäß nicht komplett abgesperrt werden kann. Zum Beispiel eignen sich eckige Funktionselemente oder Funktionselemente mit ausgeprägten durchgängigen Löchern. Die Funktionselemente werden außerdem nach Möglichkeit endständig, d.h. an einem oder beiden Enden in Längsrichtung der Endoprothese, angeordnet, da in diesem Fall die Endpunkte der Endoprothese im Röntgenbild einfach erfasst werden können.

Die Erfindung wird nachfolgend anhand von in Figuren dargestellten Ausführungsbeispielen näher erläutert. Dabei bilden alle beschriebenen und/oder bildlich dargestellten Merkmale den Gegenstand der Erfindung, unabhängig von ihrer Zusammenfassung in den Ansprüchen oder deren Rückbeziehung.

Es zeigen schematisch:
- Fig. 1: einen Längsschnitt eines ersten Ausführungsbeispiels einer Endoprothese,
- Fig. 2: einen Querschnitt durch das Ausführungsbeispiel gemäß Figur 1,
- Fig. 3: ein Funktionselement für ein Ausführungsbeispiel einer erfindungsgemäßen Endoprothese in einer perspektivischen Ansicht von der Seite,
- Fig. 4: das Ausführungsbeispiel einer erfindungsgemäßen Endoprothese in einer Ansicht von der Seite,
- Fig. 5: ein drittes Ausführungsbeispiel einer Endoprothese in einer Ansicht von der Seite,
- Fig. 6: ein Funktionselement für ein viertes Ausführungsbeispiel einer Endoprothese in einer perspektivischen Ansicht von der Seite, und
- Fig. 7: das vierte Ausführungsbeispiel einer Endoprothese in einer Ansicht von der Seite.

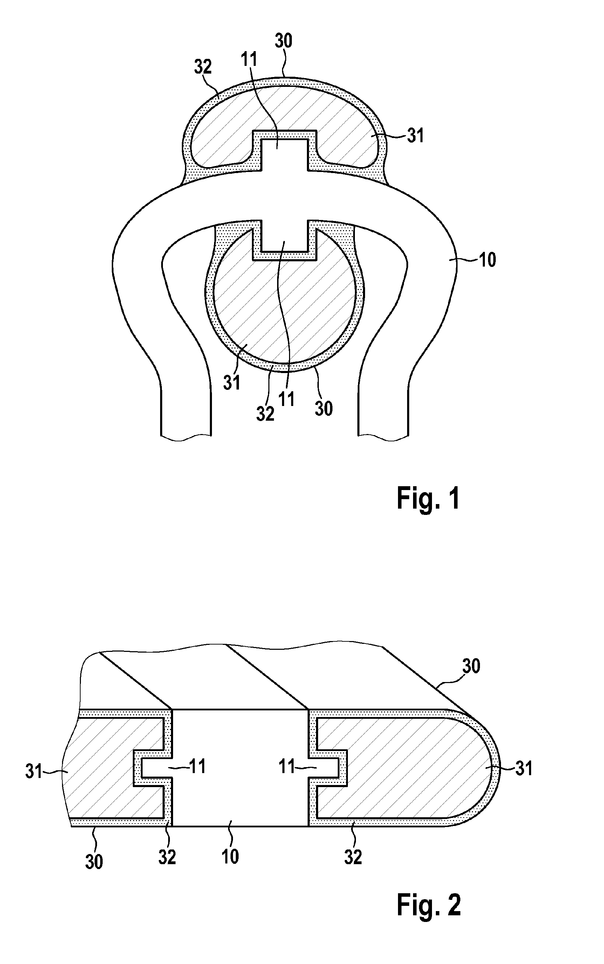

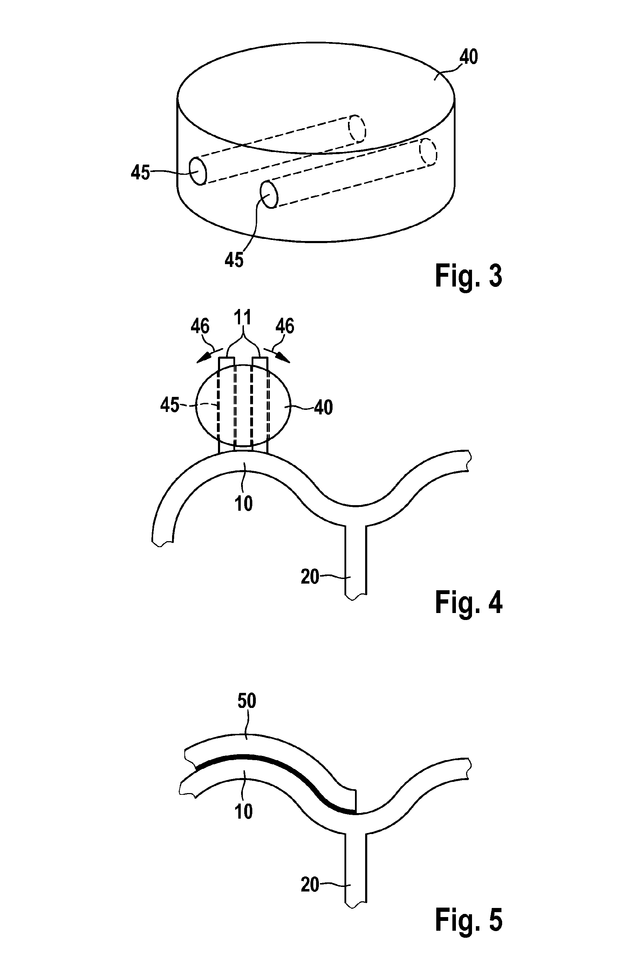

Figur 1 zeigt einen Abschnitt eines als Gitter ausgebildeten Grundkörpers eines ersten Ausführungsbeispiels einer Endoprothese, die als Stent ausgebildet ist. Das Grundkörper weist zick-zack- oder mäanderförmig gefaltete, im Wesentlichen in Umfangsrichtung verlaufende oder helixförmige Stege als Stützelemente 10 sowie im Wesentlichen in Längsrichtung des Stents verlaufende Stege als Verbindungselemente 20 (siehe Fig. 4) auf. Der in Figur 1 dargestellte Abschnitt ist ein Teil eines Stützelements 10. Der Stent ist insgesamt als eine in Richtung der Verbindungsstege 20 verlaufende rohrförmige oder hohlzylinderförmige Endoprothese, die an ihren Enden offen ist, ausgebildet.

Das Stützelement 10 weist im Bereich eines im Wesentlichen kreisförmigen Abschnitts in Längsrichtung des Stents verlaufende fingerförmige Abschnitte 11 auf. An jedem fingerförmigen Abschnitt 11 ist ein Funktionselement 30 angeordnet, das einen Kern 31 bestehend aus röntgensichtbarem (röntgenopakem) Material, wie Platin, Iridium, Gold, Wolfram, Tantal, Yttrium, Zirkon, Ytterbium oder Legierungen aus diesen Metallen oder einer Jodverbindung aufweist. Der Kern 31 des Funktionselements 30 ist vollständig umgeben von einer Barriereschicht 32, welche beispielsweise aus Parylene C besteht.

Hierbei ist die Barriereschicht 32 insbesondere derart angeordnet, dass sie zwischen dem das metallische Grundmaterial enthaltenden Kern 31 und dem Stützelement 10 bzw. zwischen dem Kern 31 und dem fingerförmigen Element 11 angeordnet ist und Kern und Stützelement 10 bzw. fingerförmiges Element 11 von einander elektrisch isoliert. Das Material der Barriereschicht 32 ist außerdem derart gestaltet, dass es biokompatibel ist. In dem in den Figuren 1 und 2 dargestellten Ausführungsbeispiel ist das Funktionselement 30 mittels einer Klebeverbindung an dem Stützelement 10 bzw. den fingerförmigen Elementen 11 befestigt.

Figur 3 zeigt ein im Wesentlichen zylinderförmiges Funktionselement 40, das zwei durchgehende, zylinderförmige Öffnungen 45 aufweist. Das Funktionselement 40 ist analog zu den in Figur 1 und 2 dargestellten Funktionselementen 30 vollständig von einer Barriereschicht umgeben. Zudem ist die Barriereschicht derart angeordnet, dass sie die durchgehenden Öffnungen vollständig umgibt. Beispielsweise bedeckt die Barriereschicht hierfür die zylindermantelförmigen Innenflächen der Öffnungen 45 vollständig.

Figur 4 zeigt, wie das Funktionselement 40 an einem Stützelement 10 angeordnet werden kann. Das Stützelement 10 weist hierfür zwei fingerförmige Elemente 11 auf, welche aus Material des Grundkörpers bestehen und welche in Längsrichtung des Stents von dem Stützelement 10 weg ragen. Diese sind drehbar und/oder biegbar ausgebildet. Nachdem das Funktionselement 40 mit einer Barriereschicht versehen wurde (siehe Erläuterungen zu Figur 3) wird es nun derart auf den fingerförmigen Elementen 11 angeordnet, dass diese in den Öffnungen 45 zu liegen kommen. Durch eine Drehbewegung um ihre Achse und/oder eine leichte Verbiegung nach außen (siehe Pfeile 46) der fingerförmigen Elemente 11 wird das Funktionselement 40 an dem Stent befestigt. Bei Verwendung der Verbiegung erfolgt die Befestigung kraftschlüssig.

Das in Figur 5 dargestellte, weitere Ausführungsbeispiel einer Endoprothese zeigt einen Abschnitt des Stents mit einem Stützelement 10 und einem Verbindungssteg 20, wobei das Stützelement 10 mit einem im Wesentlichen drahtförmigen Funktionselement 50 versehen ist. Das Funktionselement 50 weist einen im Wesentlichen zylindrischen Kern aus einem röntgenopaken Material auf, der von einer Barriereschicht vollständig umgeben ist. Das drahtförmige Funktionselement 50 ist beispielsweise mittels einer Klebeverbindung an dem Stützelement 10 des Stents befestigt. Hierbei folgt das drahtförmige Funktionselement der Form des Stützelements 10. Hierbei ist es, um das Crimpen und/oder Dilatieren der Endoprothese - falls dies erforderlich ist - nicht zu behindern, vorteilhaft, wenn das drahtförmige Funktionselement 50 an dem Stützelement 10 lediglich mittels einer Punktklebeverbindung befestigt ist und/oder wenn das drahtförmige Funktionselement 50 aus einem sehr leicht verbiegbaren, z.B. einem entsprechend dünnen, Material besteht.

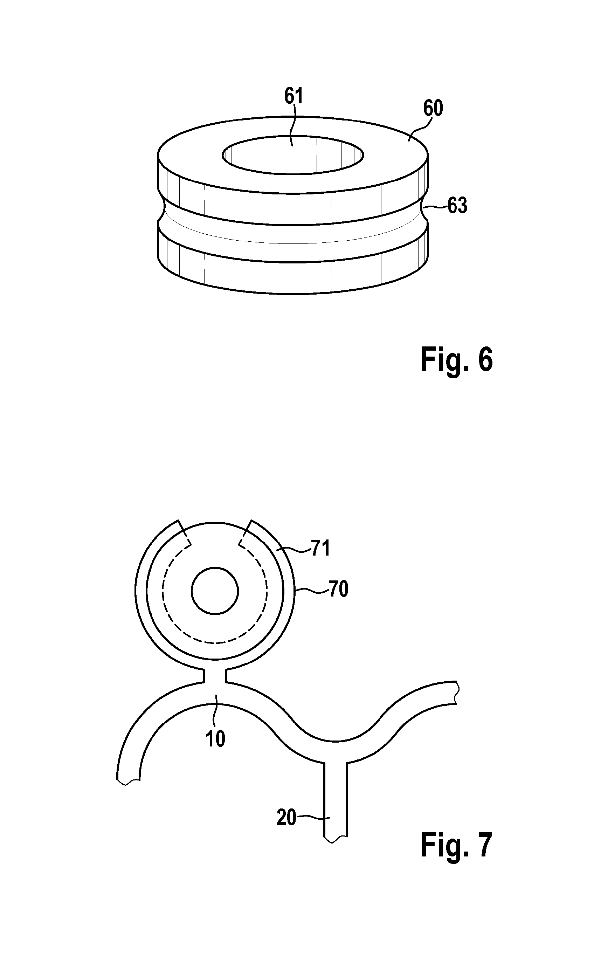

Figur 6 zeigt das Funktionselement 60 für ein weiteres Ausführungsbeispiel einer Endoprothese. Das Funktionselement 60 ist als Hohlzylinder mit einem röntgenopaken Kern und einer den Kern vollständig umgebenden Barriereschicht ausgebildet, wobei das Funktionselement 60 eine durchgehende zylindrische Öffnung 61 aufweist. Auf der Mantelfläche des Funktionselements 60 ist eine rundum laufende Nut 63 angeordnet, die zur Befestigung des Funktionselements 60 an dem Stent dient. In einem weiteren Ausführungsbeispiel kann das Funktionselement 60 ohne durchgehende Öffnung 61 verwendet werden.

Diese Befestigung an einem Stützelement 10 des Stents ist in Figur 7 dargestellt. An dem Stützelement 10 ist ein gabelförmiges Element 70 ausgebildet, das zur Aufnahme des Funktionselements 60 dient. Das gabelförmige Element 70 bildet einen ringförmigen Abschnitt 71 aus, der bei der Befestigung des Funktionselements 60 an dem Stent in der Nut 63 angeordnet wird. Zusätzlich kann durch die Anbringung einer od. mehrerer Punktklebeverbindungen auch beim Track eine sichere Befestigung des Funktionselements 60 an dem Stent erreicht werden.

Die vorstehend erläuterten Ausführungsbeispiele für eine Befestigung der Funktionselemente an einer Endoprothese in Form eines Stents zeigen lediglich einige Möglichkeiten, wie ein Funktionselement mit einer Barriereschicht in einfacher Weise mit dem Grundkörper verbunden werden kann.

### Bezugszeichenliste

- 10: Stützelement
- 11: fingerförmiger Abschnitt
- 20: Verbindungssteg
- 30: Funktionselement
- 31: Kern
- 32: Barriereschicht
- 40: Funktionselement
- 45: zylinderförmige Öffnung
- 50: Funktionselement
- 60: Funktionselement
- 61: zylindrische Öffnung
- 63: Nut
- 70: gabelförmiges Element
- 71: ringförmiger Abschnitt

## Patentansprüche

1. Endoprothese, insbesondere intraluminale Endoprothese, z.B. ein Stent, mit einem Grundkörper (10, 20), der zumindest teilweise aus einem metallischen Material besteht, und mit einem an dem Grundkörper (10, 20) befestigten oder in dieses eingebetteten Funktionselement (30, 40, 50, 60), das mindestens teilweise radioopakes und/oder röntgenopakes Material aufweist, das zumindest in einem Teil seines Volumens eine verglichen mit dem Material des Grundkörpers (10, 20) verschiedene Materialzusammensetzung aufweist, und das mit einer Barriereschicht (32) versehen ist, mittels der das radioopake und/oder röntgenopake Material (31) gegenüber dem Grundkörper (10, 20) elektrisch isoliert ist, wobei die Barriereschicht (32) das radioopake oder röntgenopake Material (31) vollständig umschließt, **dadurch gekennzeichnet, dass** das Funktionselement (40, 60) zwei durchgehende Öffnungen (45, 61) zur Befestigung des Funktionselements (40, 60) am Grundkörper (10) aufweist.

2. Endoprothese nach einem der vorhergehenden Ansprüche, **dadurch gekennzeichnet, dass** die Barriereschicht (32) eine oder mehrere Materialien aus der Gruppe enthaltend Parylene, vorzugsweise Parylene C, Polyzene F, Silikon, Gycocalix, DLC (diamond-like carbon), Cyanacrylate, insbesondere Methyl-2-Cyanoacrylat, n-Butylcyanacrylat und 2-Octyl-Cyanarylat, Titandioxid und weitere nichtleitende, permanente hydrophobe Polymere auf.

3. Endoprothese nach einem der vorhergehenden Ansprüche, **dadurch gekennzeichnet, dass** die Barriereschicht (32) ein Volumen des radio- und/oder röntgenopaken Materials (31) mit einer Querschnittsfläche eines oder mehrerer nebeneinander angeordneter Funktionselemente von mindestens etwa 200 µm x etwa 100 µm und/oder mit einer Ausdehnung in eine Richtung von mindestens etwa 300 µm und/oder einer Ausdehnung, die zwei Pixeln Auflösung von Angiographigeräten entspricht, umschließt.

4. Endoprothese nach einem der vorhergehenden Ansprüche, **dadurch gekennzeichnet, dass** das Funktionselement (30, 40, 50, 60) mit Barriereschicht (32) mittels einer Formschluss-Verbindung und/oder einer Klebeverbindung mit dem Grundkörper (10, 20) verbunden ist.

5. Endoprothese nach einem der vorhergehenden Ansprüche, **dadurch gekennzeichnet, dass** der Grundkörper (10, 20) ein biodegradierbares Material enthält, vorzugsweise Magnesium und/oder eine Magnesiumlegierung.

6. Endoprothese nach einem der vorhergehenden Ansprüche, **dadurch gekennzeichnet, dass** das Funktionselement (60) eine umlaufende Nut (63) zur Befestigung des Funktionselements (60) am Grundkörper (10) aufweist.

7. Endoprothese nach einem der vorhergehenden Ansprüche, **dadurch gekennzeichnet, dass** das Funktionselement (50) mit der umgebenden Barriereschicht drahtförmig ausgebildet ist.

## Claims

1. An endoprosthesis, in particular an intraluminal endoprosthesis, e.g. a stent, comprising a body (10, 20) consisting at least partially of a metallic material, and comprising a functional element (30, 40, 50, 60), which is fastened on the body (10, 20) or is embedded therein and which at least partially includes radiopaque and/or x-ray opaque material having, at least in a portion of the volume thereof, a material composition which differs from the material of the body (10, 20), and which is provided with a barrier layer (32), by means of which the radiopaque and/or x-ray opaque material (31) is electrically insulated with respect to the body (10, 20), wherein the barrier layer (32) completely encloses the radiopaque or x-ray opaque material (31), **characterized in that** the functional element (40, 60) has two continuous openings (45, 61) for fastening the functional element (40, 60) on the body (10).

2. The endoprosthesis according to any one of the preceding claims, **characterized in that** the barrier layer (32) comprises one or more materials from the group containing Parylene, preferably Parylene C, Polyzene F, silicone, glycocalyx, DLC (diamond-like carbon), cyanoacrylates, in particular methyl-2-cyanoacrylate, n-butyl cyanoacrylate and 2-octyl-cyanoacrylate, titanium dioxide, and other non-conductive, permanent, hydrophobic polymers.

3. The endoprosthesis according to any one of the preceding claims, **characterized in that** the barrier layer (32) encloses a volume of the radiopaque and/or x-ray opaque material (31) having a cross-sectional area of one or more functional elements disposed next to one another of at least approximately 200 µm x approximately 100 µm and/or having an expansion in one direction of at least approximately 300 µm and/or an expansion corresponding to two pixels of resolution of angiography devices.

4. The endoprosthesis according to any one of the preceding claims, **characterized in that** the functional element (30, 40, 50, 60) having a barrier layer (32) is connected to the body (10, 20) by means of an interlocking connection and/or an adhesive connection.

5. The endoprosthesis according to any one of the preceding claims, **characterized in that** the body (10, 20) contains a biodegradable material, preferably magnesium and/or a magnesium alloy.

6. The endoprosthesis according to any one of the preceding claims, **characterized in that** the functional element (60) has a peripheral groove (63) for fastening the functional element (60) on the body (10).

7. The endoprosthesis according to any one of the preceding claims, **characterized in that** the functional element (50) having the enclosing barrier layer is designed in the shape of a wire.

## Revendications

1. Endoprothèse, notamment endoprothèse intraluminale, par exemple stent, avec un corps de base (10, 20) qui est constitué au moins partiellement d'un matériau métallique, et avec un élément fonctionnel (30, 40, 50, 60) fixé sur le corps de base (10, 20) ou enseveli dans celui-ci, qui présente un matériau au moins partiellement radio-opaque et/ou opaque aux rayons X, qui présente une composition de matière différente par rapport au matériau du corps de base (10, 20) au moins sur une partie de son volume, et qui est muni d'une couche faisant barrière (32), au moyen de laquelle le matériau (31) radio-opaque et/ou opaque aux rayons X est isolé électriquement par rapport au corps de base (10, 20), où la couche faisant barrière (32) entoure totalement le matériau (31) radio-opaque et/ou opaque aux rayons X, **caractérisée en ce que** l'élément fonctionnel (40, 60) présente deux orifices (45,61) traversants pour la fixation de l'élément fonctionnel (40, 60) sur le corps de base (10).

2. Endoprothèse selon l'une des revendications précédentes, **caractérisée en ce que** la couche faisant barrière (32) présente un ou plusieurs matériaux du groupe contenant des parylènes, de préférence du Parylène C, du Polyzène F, du silicone, du Gycocalix, du carbone amorphe DLC (diamond-like carbon), des cyanoacrylates, notamment du méthyl-2-cyanoacrylate, du n-butylcyanoacrylate et du 2-octyl-cyanoacrylate, de l'oxyde de titane et d'autres polymères non conducteurs, hydrophobe permanents.

3. Endoprothèse selon l'une des revendications précédentes, **caractérisée en ce que** la couche faisant barrière (32) entoure un volume du matériau (31) radio-opaque et/ou opaque aux rayons X avec une surface transversale d'un ou de plusieurs éléments fonctionnels agencés les uns à côté des autres sur au moins environ 200 µm x environ 100 µm, et/ou avec une extension dans une direction d'au moins environ 300 µm, et/ou une extension qui correspond à deux pixels de résolution d'appareils d'angiographie.

4. Endoprothèse selon l'une des revendications précédentes, **caractérisée en ce que** l'élément fonctionnel (30, 40, 50, 60) est relié, avec la couche faisant barrière (32), au corps de base (10, 20) au moyen d'une liaison par complémentarité des formes et/ou une liaison par collage.

5. Endoprothèse selon l'une des revendications précédentes, **caractérisée en ce que** le corps de base (10, 20) contient un matériau biodégradable, de préférence, du magnésium et/ou un alliage de magnésium.

6. Endoprothèse selon l'une des revendications précédentes, **caractérisée en ce que** l'élément fonctionnel (60) présente une rainure circonférentielle (63) pour la fixation de l'élément fonctionnel (60) sur le corps de base (10).

7. Endoprothèse selon l'une des revendications précédentes, **caractérisée en ce que** l'élément fonctionnel (50) est formé en forme de fil avec la couche faisant barrière l'entourant.
